Europäisches Patentamt

European Patent Office

Office européen des brevets

(11) Veröffentlichungsnummer: **0 025 214**
**B1**

(12) # EUROPÄISCHE PATENTSCHRIFT

(45) Veröffentlichungstag der Patentschrift:
18.04.84

(21) Anmeldenummer: 80105250.7

(22) Anmeldetag: 03.09.80

(51) Int. Cl.³: **A 61 B 10/00, A 61 M 5/00**

(54) Vorrichtung zum Punktieren von körperinternen Organen, Gefässen und anderen Körpergebieten.

(30) Priorität: 07.09.79 DE 2936259

(43) Veröffentlichungstag der Anmeldung:
18.03.81 Patentblatt 81/11

(45) Bekanntmachung des Hinweises auf die Patenterteilung:
18.04.84 Patentblatt 84/16

(84) Benannte Vertragsstaaten:
AT CH DE FR GB LI NL

(56) Entgegenhaltungen:
DE - A - 2 919 024
US - A - 4 058 114

AMERICAN JOURNAL OF OBSTETRICS &
GYNECOLOGY, Band 114, Nr. 5, 1. November 1972, (US)
J. BANG et al.: "A new ultrasonic method for
transabdominal amniocentesis", Seiten 599-601
ELECTROMEDIA (SIEMENS), Band 42, Nr. 3, 1974
ERLANGEN (DE) W. JONATHA: "Amniozentese in der
Frühschwangerschaft unter Sichtkontrolle mit
Ultraschall", Seiten 94-96
ULTRASONIC, Band 10, Nr. 2, März 1972,
GUILDFORD (GB)/ H.H. HOLM et al.: "Ultrasound as a
guide in percutaneous puncture technique", Seiten
83-86

(73) Patentinhaber: SIEMENS AKTIENGESELLSCHAFT,
Berlin und München Wittelsbacherplatz 2,
D-8000 München 2 (DE)

(72) Erfinder: Haerten, Rainer, Dr. Dipl.-Phys., Auf der
Höh 13, D-8551 Röttenbach (DE)

## Vorrichtung zum Punktieren von körperinternen Organen, Gefässen und anderen Körpergebieten

Die Erfindung bezieht sich auf eine Vorrichtung zum Punktieren von körperinternen Organen, Gefässen und anderen Körpergebieten unter Verwendung einer Punktionskanüle sowie eines Ultraschall-Echo-Schnittbildgerätes mit einem Ultraschall-Applikator mit Ultraschall-Abtastsystem für die flächenweise Ultraschallabtastung des zu punktierenden Körpergebietes und einem Sichtgerät zum Sichtbarmachen des Echo-Schnittbildes, wobei dem Sichtgerät eine Einstechzielhilfe zugeordnet ist, wobei am Ultraschall-Applikator eine Führungshilfe zum Einführen der Punktionskanüle in das zu punktierende Körpergebiet in einer gewünschten Zielrichtung unter einem einstellbaren Zielwinkel vorgesehen ist, und wobei als Einstechzielhilfe eine Schaltungsanordnung zur Erzeugung und Einblendung eines Leitstrahles in das Echosichtbild am Sichtgerät dient, welcher Leitstrahl im Sichtbild die Zielrichtung der Punktionskanüle auf das zu punktierende Körpergebiet markiert.

Im Stand der Technik sind bereits eine ganze Reihe von Vorrichtungen bekannt, die bei der Feinnadelpunktion ein treffsicheres Einstechen der Punktionskanüle in ein angezieltes Organ oder Gefäss erleichtern sollen. Die meisten von ihnen arbeiten unter Zuhilfenahme von Ultraschall. So ist beispielsweise aus der US-A-3 721 227 ein Schallkopf mit zentralem Bohrloch als Führungshilfe für eine Punktionskanüle bekannt. Diese Punktionsvorrichtung erlaubt jedoch keine Punktionskontrolle im Echtzeit-Echobild. Eine solche Kontrolle ist hingegen bei einem Ultraschall-Applikator mit zentraler Schrägschlitzführung gegeben, wie er beispielsweise in der US-A-4 029 084 beschrieben ist. Die Führungsbohrung mündet jedoch in der Reihe der Ultraschallwandlerelemente; da wenigstens das zentrale Wandlerelement ausgespart werden muss, kann es zu Empfindlichkeitsverlusten im Zentrum kommen. Weitere Möglichkeiten der Punktionshilfe offenbaren die US-A-3 556 079 und die DE-C-2 148 700. Die Punktion ist hier jedoch auf Gefässe oder Organe mit strömendem Blut beschränkt, so dass die Ultraschall-Doppler-Technik ohne Schnittbild-Aufbau zur Ortung zu Hilfe genommen wird. In diesen Fällen bleibt also der Einstechvorgang einer Beobachtung im Echt-Zeitbild verschlossen.

Eine weitere Möglichkeit der Punktion ist durch den Bericht «A Trephine Needle For Vertebrae Body Biopsy» in der Zeitschrift «Lancet» vom 27. Februar 1960, Seite 35, bekannt. Hier wird unter Kontrolle im Röntgensichtbild auf der Oberfläche des Körpers, in dessen Innern ein Organ, Gefäss oder dgl. punktiert werden soll, ein Massstab angelegt, der an einem Ende eine mechanische Führungshilfe für eine Punktionskanüle trägt, die unter einem festgelegten Winkel von 55° geneigt ist. Durch Verschiebung des Massstabes auf die dem Sichtbild zu entnehmenden Abstände bei vorgegebenem Winkel kann die Einstechstelle festgelegt werden.

Demgegenüber ist in der DE-A-2 443 558 (= US-A-4 058 114) wieder eine Punktionsvorrichtung beschrieben, die mit einem Ultraschall-Echo-Schnittbildgerät arbeitet. Diese Punktionsvorrichtung umfasst eine Führungshilfe für die Punktionskanüle, die seitlich am Ultraschall-Applikator angeordnet ist. Am Sichtgerät ist ebenfalls seitlich ein mechanischer Zielstift angebracht, der zum Anzielen auf ein Punktionsgebiet im Echo-Schnittbild am Bildschirm des Sichtgerätes einstellbar ist. Der Zielwinkel des Zielstiftes lässt sich als Einstechwinkel auf die Führungshilfe für die Punktionskanüle übertragen. Die gesamte Anordnung hat den besonderen Vorteil, dass unter Echtzeitkontrolle im Ultraschall-Echoschnittbild auch seitwärts von der eigentlichen Auflagefläche des Ultraschall-Applikators eingestochen werden kann. Weniger vorteilhaft ist jedoch, dass auch am Sichtgerät selbst ein entsprechender mechanischer Aufbau als Zielhilfe benötigt wird, der relativ sperrig und zudem in der Einstellung doch zeitraubend und umständlich ist. Deshalb ist in dieser Druckschrift auch erwähnt, dass die Ablese- und Einstellelemente auch durch entsprechende elektronische Äquivalente ersetzt werden können, beispielsweise durch Widerstandspotentiometer, optoelektrische Einrichtungen wie Fotoabtaster oder dgl., die die Dreh- oder Längenänderungen des Zielstiftes in entsprechende elektrische Signale umsetzen. Der Führungshilfe sind dann von den elektrischen Signalen betätigte Steuerglieder zur selbsttätigen sinngerechten Einstellung von Zielrichtung und gegebenenfalls auch Einstellung an der Punktionskanüle zugeordnet. Es ist festzuhalten, dass bei dieser Punktionsvorrichtung — umgekehrt zur vorliegenden Erfindung — dem Zielstift (also der Zielhilfe) die besagten elektrischen Ableseelemente zugeordnet sind, die dessen Positionsänderungen in die elektrischen Signale umwandeln, anhand derer dann die Führungshilfe selbsttätig auf die Richtung des Zielstiftes eingestellt wird. Die mechanische Nachführung ist mit Zeitverzögerung verbunden. Die elektronische Einblendung einer Linie in das Echosichtbild ist hier nicht vorgesehen.

Die eingangs genannte Vorrichtung zum Punktieren geht von einer Punktionsvorrichtung aus, die in der Zeitschrift «Electromedica», Bd. 42, Nr. 3, 1974, Erlangen, W. Jonatha: «Amniozentese in der Frühschwangerschaft unter Sichtkontrolle mit Ultraschall», Seiten 94-96, beschrieben wurde. Bei dieser Vorrichtung zur Punktion unter Sicht mit einem Compound-Ultraschall-Scanner wird unter manueller Schallkopfführung ein zweidimensionales Schnittbild auf einer Speicherröhre aufgezeichnet. Die Punktion erfolgt durch einen speziellen kleinen Biopsieschallkopf mit zentraler Bohrung. Die Richtung des Schallkopfes und damit auch der Biopsienadel oder -kanüle wird durch eine elektronische Linie im Speicherbild markiert. Bereits in diesem Artikel ist angegeben, dass ein gewisser Nachteil dieser Vorrichtung darin besteht, dass die Aufnahme einen Zustand, der bereits eine gewisse Zeit zurückliegt, und nicht die aktuelle Situation während der Punktion wiedergibt.

Dies liegt daran, dass bei der Punktion mit gespeicherten B-Bildern die Position der Kanüle über ein gleichzeitig mitgeführtes A-Bild kontrolliert wird.

Aufgabe vorliegender Erfindung ist es, eine Vorrichtung der eingangs genannten Art dahingehend weiterzubilden, dass sie automatisch im Echtzeit-Verfahren eine Positionskontrolle der Punktionskanüle ermöglicht.

Diese Aufgabe wird erfindungsgemäss dadurch gelöst, dass die Führungshilfe für die Punktionskanüle am Ultraschall-Applikator einen Winkelerfasser umfasst, der in Abhängigkeit vom jeweils eingestellten Zielwinkel mit einem entsprechenden Positioniersignal den Leitstrahl im Sichtbild des Sichtgerätes auf die an der Führungshilfe eingestellte Zielrichtung einstellt.

Der Leitstrahl lässt sich bekanntlich elektronisch in einfacher Weise erzeugen und bei Bedarf in ebenso einfacher Weise in der Richtung verstellen. Der Winkelerfasser steuert diesen Leitstrahl. Das gibt den Vorteil, dass bereits mit dem Schwenken der Führungshilfe gleichzeitig auch schon der Leitstrahl geschwenkt wird. In dem Augenblick, in dem der Leitstrahl das zu punktierende Körpergebiet erreicht hat, was durch einfache Beobachtung auf dem Bildschirm festgestellt werden kann, liegt die Punktionskanüle bereits in der richtigen Einstechrichtung. Sie kann dann sofort unter weiterer Sichtkontrolle eingestochen werden.

Da die Erzeugung und Einblendung des Leitstrahles in Koordination mit der tatsächlichen Einstechrichtung der Punktionskanüle in Richtung auf das zu punktierende Objekt geschehen muss, empfiehlt es sich, in vorteilhafter Ausgestaltung der Erfindung im Ultraschall-Echo-Schnittbildgerät in der Schaltungsanordnung zur Erzeugung und Einblendung des Leitstrahles eine Verrechnungsschaltung vorzusehen, die in Abhängigkeit von den Zeilentaktsignalen eines Zeilenkippgenerators des Sichtgerätes sowie gesteuert vom Kippsignal des Bildkippgenerators desselben Sichtgerätes sowie einem Winkelsignal für die Einstechwinkelrichtung fortlaufend für den jeweiligen Bildaufbau Helltastsignale erzeugt, die in zeitlicher Aufeinanderfolge sich zum Leitstrahl zusammensetzen. Dabei kann, falls erwünscht, durch ein spezielles Selektierglied für die Helltastimpulse die Auftastung entlang des Leitstrahles zu solchen Zeitpunkten gewählt werden, dass sich Helltastpunkte für den Leitstrahl immer nur in bestimmten Abständen (z.B. in 5-mm-Abständen) voneinander ergeben. Der Leitstrahl weist dann die Form einer elektronischen Massstabsskala auf, an der sich der Tiefenabstand des zu punktierenden Objektes von der Oberfläche, auf der der Applikator liegt, ohne Schwierigkeiten sofort ablesen lässt. In Kombination mit einem verschiebbaren Reiter, wie er z.B. auch schon aus der DE-C-2 148 700 für Dopplermessungen vorbekannt ist, kann dann auch der Einstechweg und Endpunkt des Einstechweges der Punktionskanüle auf einfache Wese fortlaufend kontrolliert und festgestellt werden.

Weitere Vorteile und Einzelheiten der Erfindung ergeben sich aus der nachfolgenden Beschreibung von Ausführungsbeispielen anhand der Zeichnung in Verbindung mit den Unteransprüchen.

Es zeigen:

Fig. 1 ein erstes Ausführungsbeispiel mit Ultraschall-Array als Ultraschall-Applikator,

Fig. 2 ein zweites Ausführungsbeispiel mit Sektorabtaster als Ultraschall-Applikator,

Fig. 3 ein Prinzipschaltbild der Schaltungsanordnung im Ultraschall-Bildgerät zur Erzeugung und Einblendung des Leitstrahles.

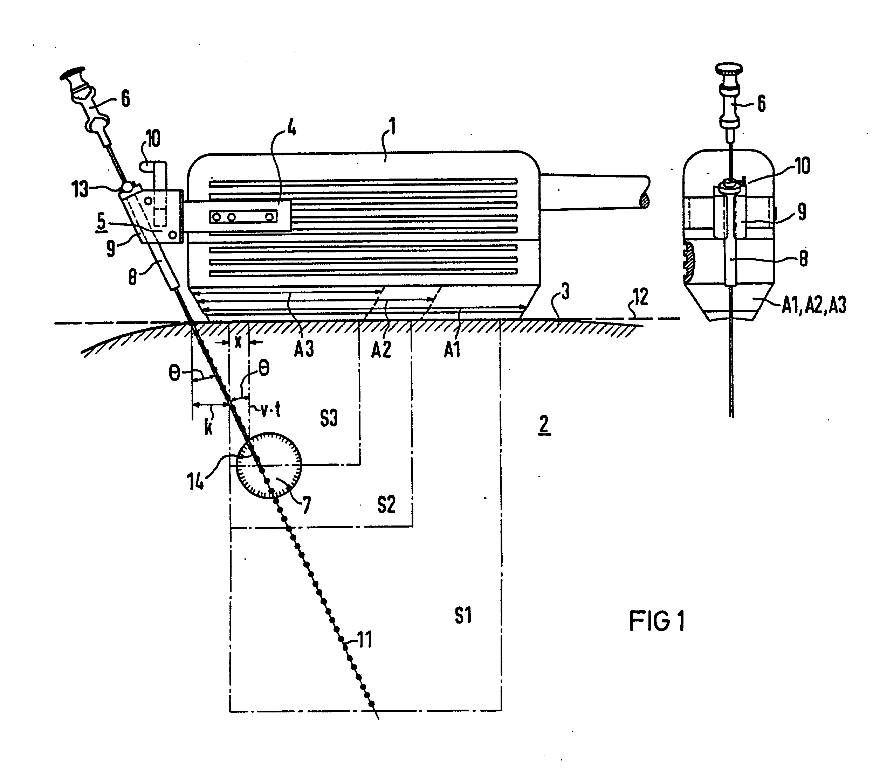

In der Fig. 1 weist der als Ultraschall-Array ausgebildete Ultraschall-Applikator 1 die aktive Elementfläche A1 auf. Das bei auf der Körperoberfläche 3 aufgesetztem Applikator im Innern des Untersuchungsobjektes 2 erzeugte Schnittbild ist S1. Wird die Punktionsvorrichtung in Verbindung mit weiteren Applikatoren mit den aktiven Elementenflächen A2 und A3 und z.B. unterschiedlicher Ultraschallfrequenz verwendet, so kann die gleiche Schaltungsanordnung verwendet werden, wenn die Applikatoren so konstruiert sind, dass die Seitenkanten der Schnittbilder S1, S2, S3 bei unterschiedlicher Grösse auf der Seite der Punktionsvorrichtung aufeinanderliegen.

Im Ausführungsbeispiel der Fig. 1 ist auf der linken Seite am Applikator 1 mittels Aufsteckbügel 4 eine Führungshilfe 5 zum Einführen einer Punktionskanüle 6 in ein Punktionsobjekt, zu punktierendes Körpergebiet oder Zielgebiet 7 im Untersuchungsobjekt 2 angeordnet. Die Führungshilfe 5 setzt sich im einzelnen zusammen aus einer Führungshülse 8 für die Punktionskanüle 6 und einer am Aufsteckbügel 4 befestigten Klammer 9 zur Halterung der Führungshülse 8. Die Klammer 9 ist mit einem Öffnungshebel 10 versehen, mit dessen Betätigung die Klammer 9 geöffnet und die Kanüle 6 samt Führungshülse 8 aus der Klammerhalterung entnommen werden kann. Bei eingestochener Punktionskanüle 6 lässt sich also durch Öffnen der Klammer 9 der gesamte Ultraschall-Applikator 1 bei Bedarf von der Punktionskanüle 6 abtrennen. Bei abgetrenntem Ultraschall-Applikator 1 kann dann ungehindert die eigentliche Biopsie durchgeführt werden.

Die in der Fig. 1 angedeuteten Schnittbilder unterschiedlicher Flächen S1, S2 und S3 lassen sich in bekannter Weise als Echo-Schnittbilder auf dem Sichtschirm einer Kathodenstrahlröhre darstellen. Auf diesem Bildschirm wird dann auch, wie in Fig. 1 angedeutet, ein Leitstrahl 11 elektronisch eingeblendet. Der Leitstrahl 11 setzt sich auf dem Sichtschirm aus einer Vielzahl von Helltastpunkten zusammen, die entlang einer Geraden angeordnet sind.

Die Helltastpunkte des Leitstrahles 11 können in bestimmten festgelegten Abständen aufeinanderfolgen, z.B. in 5-mm-Abständen, so dass der Leitstrahl 11 neben der Zielhilfe für die Punktionskanüle 6 gleichzeitig auch eine elektronische Massstabsskala bildet, an der sich der Tiefenabstand des zu punktierenden Objektes 7 von der Körperoberfläche 3, auf der der Applikator 1 liegt, ohne Schwierigkeiten sofort ablesen lässt. Gemäss der Fig. 1 liegt der elektronische Leitstrahl 11 bezüglich der Einstrahlrichtung der Ultraschallstrahlen des Ultraschall-Applikators 1 unter einem Winkel $\Theta$, der dem Winkel der Einstechrichtung für die Punktionskanüle 6 in das Objektinnere entspricht.

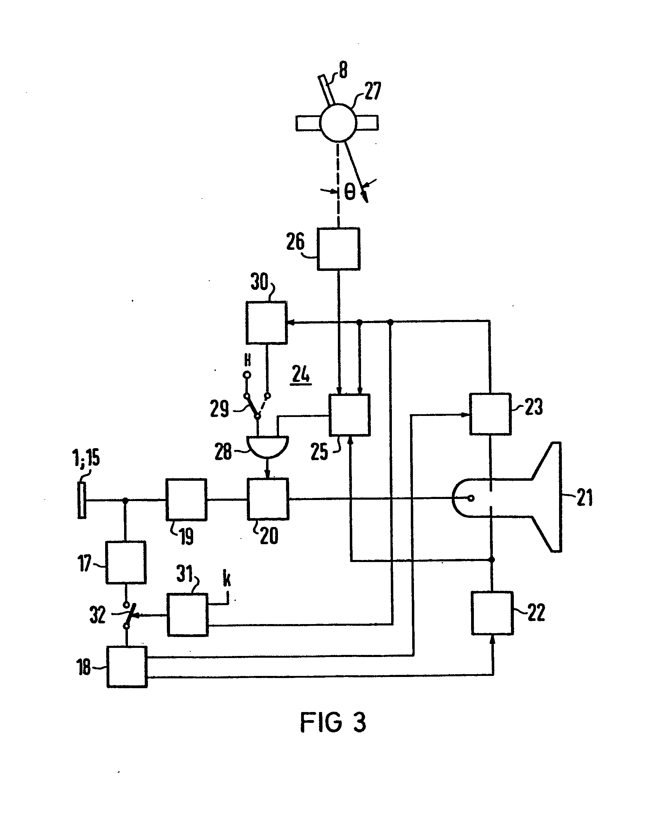

Die Koordination des Winkels $\Theta$ für Einstechrichtung und Richtung des Leitstrahles 11 erfolgt in der Weise, dass von einem Winkelerfasser 27 an der Halterung 5 der Punktionskanüle 6 ein Positioniersignal für den Leitstrahl 11 im Echosichtbild abgeleitet wird. Der Winkelerfasser 27 und seine Funktion werden in Fig. 3 noch näher erläutert. Bei Schwenken der Führungshülse 8 für die Punktionskanüle 6 wird durch dieses Positioniersignal der Leitstrahl 11 geschwenkt, so lange, bis er das angezielte Punktionsobjekt 7 erreicht. Die Punktionskanüle 11 ist dann direkt auf das Zielgebiet 7 gerichtet.

Das Einstechen erfolgt vorzugsweise unter Ultraschall-Bildkontrolle mit oder ohne Massstabeinsatz. Das Ultraschallbild der Punktionskanüle 6 ist in der Fig. 1 mit 14 bezeichnet. Das Einstechen kann aber auch blind erfolgen, wobei jedoch zumindest im letzteren Falle ein Tiefenreiter 13 angewendet werden muss, wie er aus der DE-C-2 148 700 beispielsweise vorbekannt ist. Nach Beendigung des Einstechvorganges, also dann, wenn die Spitze der Punktionskanüle 6 das Zielgebiet 7 der Biopsie erreicht hat, kann die eigentliche Entnahme von Gewebe oder Gewebsflüssigkeit für Untersuchungszwecke erfolgen. Wie vorstehend schon erwähnt, kann hierzu der Applikator 1 von der Kanüle 6 abgeklinkt werden.

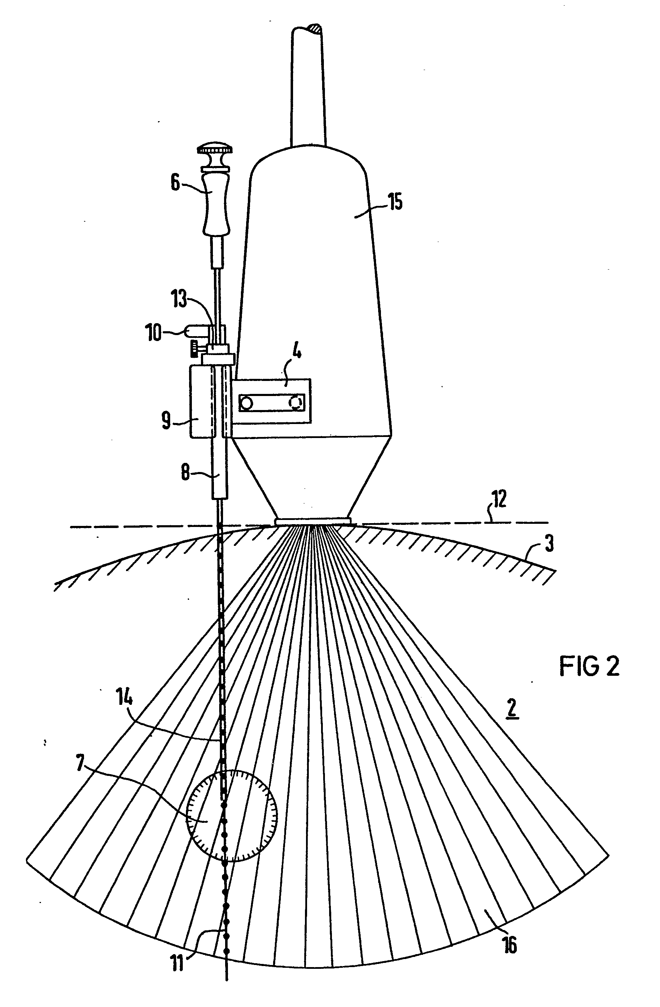

Die Anwendung des in der Fig. 1 beschriebenen Leitstrahlprinzips ist völlig unabhängig von der Ausbildungsart des Ultraschall-Applikators 1. Anstelle eines Ultraschall-Arrays kann auch ein beliebig anders gestalteter Applikator, beispielsweise ein solcher mit im Brennpunkt eines Parabelreflektors rotierendem Wandler oder ein solcher für Sektorscan, wie er in der Fig. 2 dargestellt ist, eingesetzt werden.

In Fig. 2 ist ein Sektorscanner mit 15 und das von ihm erzeugte Sektorabtastfeld mit 16 bezeichnet. Im Unterschied zur Ausführungsform der Fig. 1 ist das Führungsrohr 8 für die Punktionskanüle 6 am Sektorscanner 15 so angeordnet, dass sich, bezogen auf die Normale der Bezugsebene 12, für die Punktionskanüle 6 ein Einstechwinkel von $\Theta = 0°$ ergibt. In entsprechender Weise verläuft dann der Leitstrahl 11 ebenfalls in Richtung der Normalen, d.h. er steht senkrecht auf der Bezugsebene 12. Das Ultraschallbild der Punktionskanüle 6 ist wieder mit 14 bezeichnet.

Die Schaltungsanordnung zur Erzeugung eines Echosichtbildes mit eingeblendetem Leitstrahl 11 ist in Fig. 3 in einem Prinzipschaltbild dargestellt. Der Ultraschall-Applikator des Prinzipschaltbildes entspricht je nach Anwendungsfall dem Array 1 der Fig. 1 oder dem Sektorscanner 15 der Fig. 2 oder ist in beliebig anderer Form ausgebildet. Den Ultraschallschwingern des Applikators werden zur Aussendung von Ultraschallimpulsen Hochfrequenzimpulse eines Hochfrequenzgenerators 17 im Takte von Leitimpulsen eines Leittaktgenerators 18 zugeführt. Die empfangenen Ultraschall-Echosignale werden als elektrische Signale in einem Empfangsverstärker 19 verstärkt und der Helligkeitsmoduliereinrichtung 20 (Z-Verstärker) einer Kathodenstrahlröhre 21 zugeleitet. Der Bildaufbau erfolgt rasterförmig entsprechend dem Ultraschallabtastraster in Abhängigkeit von den Zeilenkippimpulsen eines Zeilenkippgenerators 22 und den Bildkippimpulsen eines Bildkippgenerators 23. Beide Kippgeneratoren 22, 23 werden über die Taktimpulse des Leittaktgenerators 18 gesteuert.

Die Schaltungsanordnung zur Erzeugung der Helltastimpulse für den Leitstrahl ist in Fig. 3 allgemein mit 24 bezeichnet. Diese Schaltungsanordnung 24 umfasst in erster Linie einen Rechner 25, der in Abhängigkeit vom Zeitverlauf t der Zeilenkippimpulse des Zeilenkippgenerators 22 und vom Verlauf X der Bildkippimpulse des Bildkippgenerators 23 sowie in Abhängigkeit vom Winkel $\Theta$ den Zeitpunkt für die Erzeugung eines Helltastimpulses des Leitstrahles 11 nach der folgenden Beziehung festlegt:

$$t\,(X) = \frac{X}{v tg\,\Theta} \quad \text{mit } X = k + x,$$

wobei X der Bildkipp, x die Schnittbildkoordinate und k wieder der Abstand des Auftreffpunktes der Kanüle 6 auf die Bezugsebene 12 für den Leitstrahl vom Koordinatenursprung des Schnittbildes sind. v ist die Laufgeschwindigkeit der Ultraschallwellen im Untersuchungsobjekt 2. Damit der Leitstrahl 11 vom Auftreffpunkt der Kanüle 6 auf die Bezugsebene 12 im Bild bereits sichtbar ist, beginnt also der Bildkipp X nicht erst an der linken Bildkante x = 0 der Schnittbilder, sondern bei x = - k, wobei die Sende- und Zählschaltung für das Array erst nach Durchlaufen der Strecke k gestartet wird. Die Realisierung erfolgt mittels Schalter und Komparator in der Sendersteuerschaltung (nachfolgend zu Fig. 3 noch näher beschrieben), wobei das Schalterelement vom Komparator bei $X \geqslant k$ geschlossen wird.

Der Winkel $\Theta$ ist in Abhängigkeit von der Stellung der Führungshilfe der Punktionskanüle 6 einstellbar. Hierzu erfolgt zuerst Steuerung eines Winkeleinstellgliedes 26 durch Positionssignale eines Winkelerfassungsgliedes 27 (Winkelpotentiometer) an der Führung 8 auf den Winkel der Führung und dann durch Winkelsignal des Winkeleinstellgliedes 26 Einstellung des Rechners 25 auf den gegebenen Winkel.

Die Einblendung der Hellpunkte der Leitlinie 11 in das Echosichtbild am Bildschirm der Bildröhre 21 kann Zeile für Zeile erfolgen, wodurch sich ein Leitstrahl 11 mit dicht aufeinanderfolgenden Hellpunkten ergibt. Der Leitstrahl 11 kann aber auch, wie vorstehend schon erwähnt, als Messskala eingeblendet werden. Zur Umschaltung zwischen beiden Möglichkeiten dient ein Schalter 29 in Verbindung mit einem UND-Glied 28 sowie einem weiteren Rechenglied 30. In der gezeichneten Schaltstellung des Schalters 29 liegt am zugeordneten Eingang des UND-Gliedes 28 dauernd das Potential H. Die im Zeilentakt anfallenden kurzen Ausgangsimpulse des Rechengliedes 25 gelangen durch das UND-Glied 28 zur Helltasteinrichtung 20, die daraufhin im Zeilentakt dicht aufeinanderfolgende Hellpunkte für den Leitstrahl 11 erzeugt.

Bei Umschaltung des Schalters 29 in die gestrichelte Schaltstellung werden dem betreffenden Eingang des UND-Gliedes 28 hingegen längere Ausgangsimpulse des Rechengliedes 30 zugeleitet. Diese Ausgangsimpulse werden in Zeitabständen

$$\tau = \frac{a \cdot \cos\,\Theta}{v}$$

erzeugt, wobei a das erwünschte Abstandsmass der Messskala, beispielsweise 5 mm, bedeutet. $\Theta$ ist

wieder der Zielwinkel und v die Geschwindigkeit der Ultraschallwellen im Untersuchungsobjekt. Mit den so an das UND-Glied 28 abgegebenen Impulsen ergibt sich zeitliche Übereinstimmung mit den Zeilenimpulsen des Rechengliedes 25 immer nur in bestimmten Zeitabständen $\tau$, die am Bildschirm in durch a vorgegebene Wegabstände zwischen den einzelnen Hellpunkten des Leitstrahles umgesetzt werden. Wird also a beispielsweise zu 5 mm gewählt, so setzt sich der Leitstrahl 11 am Bildschirm der Röhre 21 aus Hellpunkten zusammen, die entlang dem Leitstrahl jeweils im Abstand von a = 5 mm liegen. Der Leitstrahl entspricht also einer eingeblendeten elektronischen Messskala mit 5-mm-Abständen. Dieses Verfahren setzt ein zeilendichtes Bild voraus, wie es z.B. bei Zeilenvervielfachung durch digitale Fernsehnormwandlung entsteht. Die Länge des vom Rechner 30 gelieferten Impulses sollte mindestens gleich einer Zeilenkipp-Periode sein. In sämtlichen Einblendverfahren erfolgt die Einblendung so, dass der Leitstrahl bereits auf der linken Seite vor Beginn des eigentlichen Echoschnittbildes mit Einstechpunkt an der Bezugsebene am Sichtschirm des Bildgerätes sichtbar gemacht wird. Wie vorstehend schon angedeutet, wird der Abstand k des Einstechpunktes vom Koordinatenursprung des Bildes berücksichtigt durch Einsatz eines Komparators 31 in Verbindung mit dem Einschalter 32 für den Hochfrequenzimpulsgenertor 17. Der Schalter 32 wird durch den Komparator 31 erst immer dann geschlossen, wenn die Bildkippspannung X die Signalschwelle k erreicht bzw. überschreitet.

Modifikationen der beschriebenen Ausführungsformen sind möglich. So kann unter anderem mit oder auch alternativ zum Leitstrahl (d.h. unter dessen vorübergehender Abschaltung) zur Distanzmessung bzw. zur Messung der Punktionstiefe ein Messkreuz über eine Messeinrichtung, wie sie beispielsweise in der DE-B-2 910 022 beschrieben ist, in das Echosichtbild eingeblendet werden. Hierzu braucht lediglich im Arbeitsmode «Biopsie» auf dem Leitstrahl oder an jener Stelle, die durch Lage und Richtung des ausgeblendeten Leitstrahles vorgegeben ist, eine Messmarke an den gewünschten Zielort geführt und über die Distanzmesseinrichtung der Abstand längs des Leitstrahles bis zur Bezugsebene an der Körperoberfläche gemessen und digital angezeigt zu werden. Die zweite Messmarke ist dann automatisch auf den Einstechpunkt auf der Bezugsebene fixiert.

**Patentansprüche**

1. Vorrichtung zum Punktieren von körperinternen Organen, Gefässen und anderen Körpergebieten (7), unter Verwendung einer Punktionskanüle (6) sowie eines Ultraschall-Echo-Schnittbildgerätes mit einem Ultraschall-Applikator (1; 15) mit Ultraschall-Abtastsystem für die flächenweise Ultraschallabtastung des zu punktierenden Körpergebietes (7) und einem Sichtgerät (21) zum Sichtbarmachen des Echo-Schnittbildes, wobei dem Sichtgerät (21) eine Einstechzielhilfe zugeordnet ist, wobei am Ultraschall-Applikator (1; 15) eine Führungshilfe (5) zum Einführen der Punktionskanüle (6) in das zu punktierende Körpergebiet (7) in einer gewünschten Zielrichtung unter einem einstellbaren Zielwinkel ($\Theta$) vorgesehen ist, und wobei als Einstechzielhilfe eine Schaltungsanordnung (24) zur Erzeugung und Einblendung eines Leitstrahles (11) in das Echosichtbild (S1, S2, S3; 16) am Sichtgerät (21) dient, welcher Leitstrahl (11) im Sichtbild (S1, S2, S3; 16) die Zielrichtung der Punktionskanüle (6) auf das zu punktierende Körpergebiet (7) markiert, dadurch gekennzeichnet, dass die Führungshilfe (5) für die Punktionskanüle (6) am Ultraschall-Applikator (1; 15) einen Winkelerfasser (27) umfasst, der in Abhängigkeit vom jeweils eingestellten Zielwinkel ($\Theta$) mit einem entsprechenden Positioniersignal den Leitstrahl (11) im Sichtbild des Sichtgerätes (21) auf die an der Führungshilfe (5) eingestellte Zielrichtung einstellt.

2. Vorrichtung nach Anspruch 1, dadurch gekennzeichnet, dass im Ultraschall-Echo-Schnittbildgerät in der Schaltungsanordnung (24) zur Erzeugung und Einblendung des Leitstrahles (11) eine Verrechnungsschaltung (25) vorgesehen ist, die in Abhängigkeit von den Zeilentaktsignalen eines Zeilenkippgenerators (22) des Sichtgerätes (21) sowie gesteuert vom Kippsignal des Bildkippgenerators (23) des Sichtgerätes (21) und von einem Winkelsignal des Winkelerfassers (27) fortlaufend für den jeweiligen Bildaufbau Helltastsignale erzeugt, die in zeitlicher Aufeinanderfolge sich zum Leitstrahl (11) im Sichtbild zusammensetzen.

3. Vorrichtung nach Anspruch 2, dadurch gekennzeichnet, dass die Helltastimpulse für den Leitstrahl (11) im Zeilentakt der Kippimpulse des Zeilenkippgenerators (22) des Sichtgerätes (21) erzeugt werden.

4. Vorrichtung nach Anspruch 2, dadurch gekennzeichnet, dass Helltastimpulse für den Leitstrahl (11) immer nur zu bestimmten Zeitpunkten erzeugt werden, so dass sich Helltastpunkte für den Leitstrahl (11) immer nur in bestimmten Abständen längs des Leitstrahles voneinander ergeben, so dass dann der Leitstrahl die Form einer elektronischen Messstabskala aufweist.

5. Vorrichtung nach einem der Ansprüche 2 bis 4, dadurch gekennzeichnet, dass zur Erzeugung von Helltastsignalen für den Leitstrahl (11) in der Schaltungsanordnung (24) ein Rechner (25) vorgesehen ist, der in Abhängigkeit vom Zeitverlauf (t) der Zeilenkippimpulse des Zeilenkippgenerators (22) und vom Verlauf (X) der Bildkippimpulse des Bildkippgenerators (23) sowie in Abhängigkeit vom Zielwinkel ($\Theta$) den Zeitpunkt für die Erzeugung eines Helltastimpulses des Leitstrahles (11) nach der folgenden Beziehung festlegt:

$$t(X) = \frac{X}{v \cdot tg\,\Theta} \quad , \text{mit } X = x + k,$$

wobei X der Bildkipp, x die Schnittbildkoordinate und k der Abstand des Auftreffpunktes der Kanüle (6) auf die Bezugsebene (12) für den Leitstrahl (11) vom Koordinatenursprung des Schnittbildes sowie v die Laufgeschwindigkeit der Ultraschallwellen im Untersuchungsobjekt (2) bedeuten.

6. Vorrichtung nach Anspruch 5, dadurch gekennzeichnet, dass zur Einblendung des Leitstrahles (11) in Form einer Massstabsskala in der Schaltungsanordnung (24) dem Rechner (25) ein weiterer Rechner (30) zugeordnet ist, der Ausgangsimpulse in Zeitabständen $\tau = \dfrac{a \cos \Theta}{v}$ erzeugt, wobei a das erwünschte Abstandsmass der Messskala, beispielsweise 5 mm, bedeutet, und wobei $\Theta$ wieder der Zielwinkel und v die Geschwindigkeit der Ultraschallwellen im Untersuchungsobjekt sind.

7. Vorrichtung nach Anspruch 6, dadurch gekennzeichnet, dass beide Rechner (25 und 30) ausgangsseitig mit den Eingängen eines UND-Gliedes (28) verbindbar sind, das bei erwünschter Einblendung des Leitstrahles (11) als Messskala Ausgangsimpulse an die Helltasteinrichtung (20) für das Sichtgerät (21) nur dann abgibt, wenn zum Zeitpunkt des Anfallens von Ausgangsimpulsen des ersten Rechners (25) gleichzeitig auch Ausgangsimpulse des zweiten Rechners (30) vorhanden sind.

8. Vorrichtung nach einem der Ansprüche 1 bis 7, dadurch gekennzeichnet, dass die Nadelführung (8) der Kanülen-Führungshilfe (5) am Ultraschall-Applikator (1) mittels Klinkmitteln (9, 10) abklinkbar befestigt ist, so dass nach erfolgtem Einstechvorgang zwecks Durchführung der eigentlichen Biopsie der Applikator (1) von der Punktionskanüle (6) abgetrennt werden kann.

9. Vorrichtung nach einem der Ansprüche 1 bis 8, dadurch gekennzeichnet, dass zur Distanzmessung oder zur Messung der Punktionstiefe mittels Messmarkeneinblender im Sichtbild eine Messmarke einblendbar ist, die wahlweise bei auf- oder ausgeblendetem Leitstrahl (11) in dessen Richtung im Sichtbild an den gewünschten Zielort geführt wird, und dass über die Distanzmesseinrichtung der Abstand längs des Leitstrahles (11) bis zur Bezugsebene (12) an der Körperoberfläche (3) gemessen und vorzugsweise digital anzeigbar ist.

10. Vorrichtung nach Anspruch 9, dadurch gekennzeichnet, dass eine zweite Messmarke im Sichtbild automatisch auf den Einstechpunkt auf der Bezugsebene (12) fixiert ist.

**Claims**

1. Apparatus for puncturing internal body organs, vessels and other body areas (7) using a puncture cannula (6) and an ultrasonic device for producing echo sectional images, the device having an ultrasonic scanning system with an ultrasonic applicator (1; 15) for scanning the surface of the body area (7) which is to be punctured, and a display unit (21) for displaying the echo sectional image, with directing means associated therewith, wherein the ultrasonic applicator (1; 15) has provision for guide means (5) for inserting the puncture cannula (6) into the body area (7) which is to be punctured in a desired, aimed direction at an adjustable desired angle ($\Theta$), and wherein the directing means comprises a circuit arrangement (24) which produces and superimposes on the image (S1, S2, S3; 16) displayed on the display unit (21), a guide beam (11), which guide beam (11) in the display image (S1, S2, S3; 16) marks the desired direction of the puncture cannula (6) with respect to the body area (7) which is to be punctured, characterised in that the guide means (5) on the ultrasonic applicator (1; 15) for inserting the puncture cannula (6) comprises an angle detector (27) which adjusts the guide beam (11), in the display image of the display unit (21), in dependence on a desired angle ($\Theta$) by means of a corresponding positioning signal, to the target direction adjusted at the guide means (5).

2. Apparatus according to claim 1, characterised in that in the circuit arrangement (24) is provided a calculating circuit (25) which continuously produces intensity modulating signals as a function of horizontal time signals from a horizontal time base generator (22) of the display unit (21) and of time base signals from a vertical time base generator (23) of the display unit (21), and as a function of an angle signal from the angle detector (27), the intensity modulating signals combining in chronological order to form the guide beam (11) in the display image.

3. Apparatus according to claim 2, characterised in that the intensity modulating pulses for the guide beam (11) are produced at the line synchronisation frequency of the time base pulses from the horizontal time base generator (22) of the display unit (21).

4. Apparatus according to claim 2, characterised in that intensity modulating pulses for the guide beam (11) are always only produced at specific intervals so that intensity-modulated spots for the guide beam (11) are always only produced at specific intervals along the guide beam such that the guide beam has the form of an electronic measuring line scale.

5. Apparatus according to any one of claims 2 to 4, characterised in that, for the purpose of producing intensity modulating signals for the guide beam (11), there is provision in the switching arrangement (24) for a calculator (25) which produces the intensity modulating signal of the guide beam (11) according to the following relationship:

$$t\,(X) = \frac{X}{v \, tg \, \Theta} \quad , \text{ with } X = x + k,$$

where: $t$ is the course of horizontal time base pulses from the horizontal time base generator (22); $X$ is the course of vertical time base pulses from the vertical time base generator (23); $\Theta$ is an angle detected by the angle detector; $x$ is an image co-ordinate; $k$ is the distance of the point of intersection of the cannula (6) with a reference plane (12) for the guide beam (11) from the image co-ordinate origin; and $v$ is the sweep speed of ultrasonic waves in the body being examined (2).

6. Apparatus according to claim 5, characterised in that, for the purpose of superimposing the guide beam (11) in the form of a measuring line scale, there is provided in the switching arrangement (24) a further calculator (30), associated with the calculator (25), which producess output pulses at time intervals $\tau$, where: $\tau = \dfrac{a \cos \Theta}{v}$ ; and where $a$ is a desired distance measurement of the measuring scale, for

example 5 mm; Θ is an angle detected by the angle detector; and v is the sweep speed of ultrasonic waves in the body being examined.

7. Apparatus according to claim 6, characterised in that both calculators (25 and 30) can have a respective output connected to a respective input of an AND gate (28) which, in order to produce the desired superimposition of the guide beam (11) in the form of a measuring scale, emits output pulses to the intensity modulating device (20) of the display unit (21) only when the production of output pulses of the first calculator (25) coincides with the production of output pulses of the second calculator (30).

8. Apparatus according to any one of claims 1 to 7, characterised in that a needle guide (8) of the guide means (5) on the ultrasonic applicator (1) is secured by way of latching means (9, 10) in such a manner that it may be unlatched so that after the insertion process has taken place, for the purpose of carrying out a biopsy, the applicator 6;7 can be separated from the puncture cannula (6).

9. Apparatus according to any one of claims 1 to 8, characterised in that, for measuring distance or for measuring the depth of puncture by means of a superimposed measuring image on the display image, a measuring image can be superimposed which image is guided selectively in the direction of the superimposed or faded out guide beam (11) in the display image to the desired target area, and in that by way of a distance-measuring device the distance along the guide beam (11) to the reference plane (12) on the body surface (3) is measured and can be registered, preferably in digits.

10. Apparatus according to claim 9, characterised in that a second measuring image is automatically fixed in the display image at the point of insertion on the reference plane (12).

## Revendications

1. Dispositif pour ponctionner des organes, vaisseaux et autres parties (7) internes du corps en utilisant une canule de ponction (6) ainsi qu'un appareil de reproduction d'image en coupe d'écho ultrasons, comportant un applicateur d'ultrasons (1; 15), qui comprend un système d'exploration par ultrasons pour l'exploration par ultrasons suivant une surface de la partie (7) du corps à ponctionner, et un appareil de visualisation (21) pour rendre visible l'image en coupe d'écho, un dispositif auxiliaire de visée pour la piqûre étant associé à l'appareil de visualisation (21), et, sur l'applicateur d'ultrasons (1; 15), étant prévu un moyen auxiliaire pour introduire la canule de ponction (6) dans la partie (7) du corps à ponctionner suivant une direction de visée souhaitée avec un angle de visée réglable (Θ), et un circuit (24) destiné à produire et à surimprimer un rayon de guidage (11) dans l'image d'écho visible (S1, S2, S3; 16) sur l'appareil de visualisation (21) servant de moyen auxiliaire de visée pour la piqûre, rayon de guidage (11) qui indique sur l'image visible (S1, S2, S3; 16) la direction de visée de la canule de ponction (6) en direction de la partie (7) du corps à ponctionner, caractérisé par le fait que le moyen auxiliaire de guidage (5) pour la canule de ponction (6) sur l'applicateur d'ultrasons (1; 15) comprend un détecteur d'angle (27) qui, en fonction de l'angle de visée (Θ) respectivement réglé, règle le rayon de guidage (11) sur l'image visible de l'appareil de visualisation (21) sur la direction de visée réglée au niveau du moyen auxiliaire de guidage (5), au moyen d'un signal de positionnement correspondant.

2. Dispositif suivant la revendication 1, caractérisé par le fait que dans l'appareil de reproduction d'image en coupe d'écho d'ultrasons il est prévu, dans le circuit (24) pour produire et surimprimer le rayon de guidage (11), un dispositif de compensation (25) qui, en fonction des signaux de cadence de lignes d'une unité de base de temps de lignes (22) de l'appareil de visualisation (21) ainsi que sous la commande du signal de balayage de l'unité de base de temps d'image (23) de l'appareil de visualisation (21) et d'un signal angulaire du détecteur d'angle (27), produit en permanence pour la synthèse respective de l'image des signaux d'allumage du spot qui s'assemblent les uns à la suite des autres dans le temps pour former le rayon de guidage (11) sur l'image visible.

3. Dispositif suivant la revendication 2, caractérisé par le fait que les impulsions d'allumage du spot pour le rayon de guidage (11) sont produites avec la cadence de lignes des impulsions de balayage de l'unité de base de temps de lignes (22) de l'appareil de visualisation (21).

4. Dispositif suivant la revendication 2, caractérisé par le fait que les impulsions de balayage du spot pour le rayon de guidage (11) sont toujours produites uniquement à des instants déterminés, de sorte que des points d'allumage du spot pour le rayon de guidage (11) n'apparaissent toujours qu'à des distances déterminées le long du rayon de guidage, de manière que le rayon de guidage présente la forme d'une échelle graduée électronique.

5. Dispositif suivant l'une des revendications 2 à 4, caractérisé par le fait que pour produire des signaux d'allumage du spot pour le rayon de guidage (11), il est prévu dans le circuit (24) un calculateur (25) qui, en fonction de l'allure dans le temps (t) des impulsions de balayage de lignes de l'unité de base de temps de lignes (22) et de l'allure (X) des impulsions de balayage d'image de l'unité de base de temps d'image (23) ainsi qu'en fonction de l'angle de visée (Θ), détermine l'instant pour la production d'une impulsion d'allumage du spot du rayon de guidage (11) d'après la relation suivante:

$$t(X) = \frac{X}{v \cdot tg\,\Theta} \quad , \text{avec } X = x + k,$$

X représentant le balayage d'image, x la coordonnée de l'image en coupe et k la distance du point d'impact de la canule (6) sur le plan de référence (12) pour le rayon de guidage (11) par rapport à l'origine des coordonnées de l'image en coupe, et v la vitesse de propagation des ondes ultrasonores dans l'objet examiné (2).

6. Dispositif suivant la revendication 5, caractérisé par le fait que pour surimprimer le rayon de guidage (11) sous la forme d'une échelle graduée, dans le circuit (24) au calculateur (25) est associé un autre calculateur (30) qui produit des impulsions de sortie à des intervalles de temps $\tau = \dfrac{a \cos \Theta}{v}$, $\underline{a}$ étant la graduation souhaitée de l'échelle graduée, par exemple 5 mm, et $\Theta$ étant de nouveau l'angle de visée et v la vitesse des ondes ultrasonores dans l'objet examiné.

7. Dispositif suivant la revendication 6, caractérisé par le fait que les deux calculateurs (25 et 30) peuvent être reliés, côté sortie, aux entrées d'un élément ET (28) qui, lors d'une surimpression souhaitée du rayon de guidage (11) en tant qu'échelle graduée, délivre alors des impulsions de sortie au dispositif d'allumage du spot (20) pour l'appareil de visualisation (21) uniquement lorsque des impulsions de sortie du second calculateur (30) existent également simultanément à l'instant de l'apparition d'impulsions de sortie du premier calculateur (25).

8. Dispositif suivant l'une des revendications 1 à 7, caractérisé par le fait que le guidage (8) de l'aiguille du moyen auxiliaire de guidage (5) de la canule est fixé de façon détachable au niveau de l'applicateur (1)

d'ultrasons, à l'aide de moyens à cliquet, de sorte que l'applicateur (1) peut être séparé de la canule de ponction (6) une fois que le processus de piqûre est effectué, dans le but de réaliser la biopsie proprement dite.

9. Dispositif suivant l'une des revendications 1 à 8, caractérisé par le fait que pour la mesure de distance ou pour la mesure de la profondeur de ponction on peut surimprimer à l'image visible, au moyen d'un dispositif de surimpression de repères de mesure, un repère de mesure qui est placé au niveau du but souhaité sur l'image visible suivant la direction du rayon de guidage (11), que celui-ci soit présent ou non, et que la distance le long du rayon de guidage (11) jusqu'au plan de référence (12) au niveau de la surface (3) du corps est mesurée par l'intermédiaire du dispositif de mesure de distance, et peut de préférence être affichée numériquement.

10. Dispositif suivant la revendication 9, caractérisé par le fait qu'un second repère de mesure est fixé automatiquement sur l'image visible au niveau du point de piqûre sur le plan de référence (12).

FIG 1

0 025 214

FIG 2

FIG 3